(19) Europäisches Patentamt
European Patent Office
Office européen des brevets

(11) EP 1 103 545 B1

(12) # EUROPEAN PATENT SPECIFICATION

(45) Date of publication and mention of the grant of the patent:
**05.11.2003 Bulletin 2003/45**

(51) Int Cl.[7]: **C07D 213/75**, A61K 31/44, A61P 25/22, A61P 25/24

(21) Application number: **00125450.7**

(22) Date of filing: **21.11.2000**

(54) **2-(3,5-Bis-trifluoromethyl-phenyl)-N-methyl-N-(6-morpholin-4-yl-4-o-tolyl-pyridin-3-yl)-isobutyramide**

2-(3,5-Bis-trifluoromethyl-phenyl)-N-methyl-N-(6-morpholin-4-yl-4-o-tolyl-pyridin-3-yl)-isobutyramid

2-(3,5-Bis-trifluorométhyl-phényl)-N-méthyl-N-(6-morpholin-4-yl-4-o-tolyl-pyridin-3-yl)-isobutyramide

(84) Designated Contracting States:
**AT BE CH CY DE DK ES FI FR GB GR IE IT LI LU MC NL PT SE TR**
Designated Extension States:
**LT LV RO SI**

(30) Priority: **29.11.1999 EP 99123685**

(43) Date of publication of application:
**30.05.2001 Bulletin 2001/22**

(73) Proprietor: **F. HOFFMANN-LA ROCHE AG**
**4070 Basel (CH)**

(72) Inventors:
- **Ballard, Theresa Maria**
  **4056 Basle (CH)**
- **Higgins, Guy Andrew**
  **4054 Basle (CH)**
- **Hoffmann, Torsten**
  **4127 Birsfelden (CH)**
- **Poli, Sonia Maria**
  **4053 Basle (CH)**
- **Sleight, Andrew**
  **68400 Riedisheim (FR)**

(74) Representative: **Poppe, Regina et al**
**124 Grenzacherstrasse**
**4070 Basle (CH)**

(56) References cited:
**EP-A- 0 916 346**
**EP-A- 1 035 115**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 103 545 B1

## Description

The present invention relates to the compound of formula

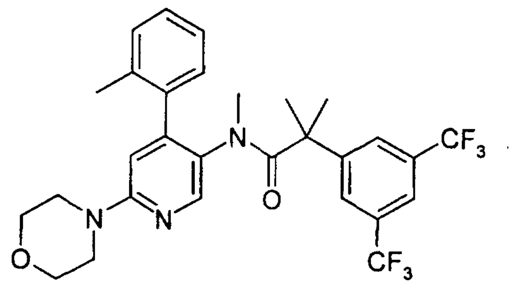

I

and to pharmaceutically acceptable acid addition salts thereof.
This compound is generically encompassed by the second priority of EP 1 035 115, which is the same priority date as of the present invention.

The compound of formula I and its salts are characterized by valuable therapeutic properties. It has been found that the compound of the present invention is a highly selective antagonist of the Neurokinin 1 (NK-1, substance P) receptor. Substance P is a naturally occurring undecapeptide belonging to the tachykinin family of peptides, the latter being so-named because of their prompt contractile action on extravascular smooth muscle tissue.
The receptor for substance P is a member of the superfamily of G protein-coupled receptors.

The neuropeptide receptor for substance P (NK-1) is widely distributed throughout the mammalian nervous system (especially brain and spinal ganglia), the circulatory system and peripheral tissues (especially the duodenum and jejunum) and are involved in regulating a number of diverse biological processes.

The central and peripheral actions of the mammalian tachykinin substance P have been associated with numerous inflammatory conditions including migraine, rheumatoid arthritis, asthma, and inflammatory bowel disease as well as mediation of the emetic reflex and the modulation of central nervous system (CNS) disorders such as Parkinson's disease (Neurosci. Res., 1996, 7, 187-214), anxiety (Can. J. Phys., 1997, 75, 612-621) and depression (Science, 1998, 281, 1640-1645).

Evidence for the usefulness of tachykinin receptor antagonists in pain, headache, especially migraine, Alzheimer's disease, multiple sclerosis, attenuation of morphine withdrawal, cardiovascular changes, oedema, such as oedema caused by thermal injury, chronic inflammatory diseases such as rheumatoid arthritis, asthma/bronchial hyperreactivity and other respiratory diseases including allergic rhinitis, inflammatory diseases of the gut including ulcerative colitis and Crohn's disease, ocular injury and ocular inflammatory diseases has been reviewed in "Tachykinin Receptor and Tachykinin Receptor Antagonists", J. Auton. Pharmacol., 13, 23-93, 1993.

Furthermore, Neurokinin 1 receptor antagonists are being developed for the treatment of a number of physiological disorders associated with an excess or imbalance of tachykinin, in particular substance P. Examples of conditions in which substance P has been implicated include disorders of the central nervous system such as anxiety, depression and psychosis (WO 95/16679, WO 95/18124 and WO 95/23798).

The neurokinin-1 receptor antagonists are further useful for the treatment of motion sickness and for treatment induced vomiting.

In addition, in The New England Journal of Medicine, Vol. 340, No. 3, 190-195, 1999 has been described the reduction of cisplatin-induced emesis by a selective neurokinin-1-receptor antagonist.

The usefulness of neurokinin 1 receptor antagonists for the treatment of certain forms of urinary incontinence is further described in Neuropeptides, 32(1), 1-49, (1998) and Eur. J. Pharmacol., 383(3), 297-303, (1999).

Furthermore, US 5,972,938 describes a method for treating a psychoimmunologic or a psychosomatic disorder by administration of a tachykinin receptor, such as NK-1 receptor antagonist.

Life Sci., (2000), 67(9), 985-1001 describes, that astrocytes express functional receptors to numerous neurotransmitters including substance P, which is an important stimulus for reactive astrocytes in CNS development, infection and injury. In brain tumors malignant glial cells originating from astrocytes are triggered by tachykinins via NK-1 receptors to release soluble mediators and to increase their proliferative rate. Therefore, selective NK-1 receptor antagonists may be useful as a therapeutic approach to treat malignant gliomas in the treatment of cancer.

In Nature (London) (2000), 405(6783), 180-183 is described that mice with a genetic disruption of NK-1 receptor show a loss of the rewarding properties of morphine. Consequently NK-1 receptor antagonists may be useful in the treatment of withdrawel symptoms of addictive drugs such as opiates and nicotine and reduction of their abuse/craving.

Objects of the present invention are the compound of formula I and pharmaceutically acceptable salts thereof, the preparation of the above-mentioned compound, medicaments containing this compound and their manufacture as well as the use of the above-mentioned compound in the control or prevention of illnesses, especially of illnesses and disorders of the kind referred to earlier or in the manufacture of corresponding medicaments.

The most preferred indications in accordance with the present invention are those, which include disorders of the central nervous system, for example the treatment or prevention of certain depressive disorders, anxiety or emesis by the administration of the NK-1 receptor antagonist. A major depressive episode has been defined as being a period of at least two weeks during which, for most of the day and nearly every day, there is either depressed mood or the loss of interest or pleasure in all, or nearly all activities.

As described therein, the term "pharmaceutically acceptable acid addition salts" embraces salts with inorganic and organic acids, such as hydrochloric acid, nitric acid, sulfuric acid, phosphoric acid, citric acid, formic acid, fumaric acid, maleic acid, acetic acid, succinic acid, tartaric acid, methanesulfonic acid, p-toluenesulfonic acid and the like.

The present compound of formula I and their pharmaceutically acceptable salts can be prepared by methods known in the art, for example, by processes described below, which process comprises

a) reacting the compound of formula

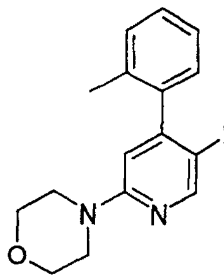

II

with the compound of formula

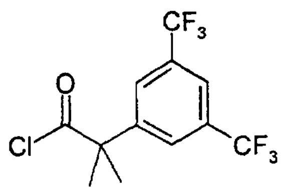

III

to the compound of formula

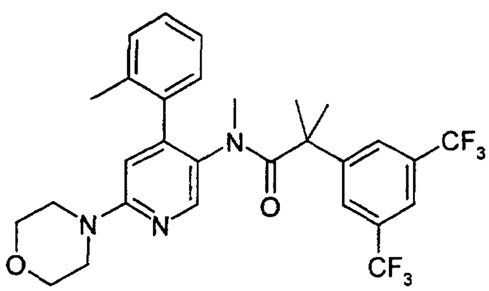

I

and,

if desired, converting the compound obtained into a pharmaceutically acceptable acid addition salt.

In accordance with process variant a) DIPEA (N-ethyldiisopropyl-amine) is added to a mixture of the compound of formula II and the compound of formula III in dichloromethane and the mixture is stirred at temperatures

between 35-40°C. The desired compound of formula I is yielded after purification in good yields.

The salt formation is effected at room temperature in accordance with methods which are known *per se* and which are familiar to any person skilled in the art. Not only salts with inorganic acids, but also salts with organic acids are possible. Hydrochlorides, hydrobromides, sulphates, nitrates, citrates, acetates, maleates, succinates, methanesulphonates, p-toluenesulphonates and the like are examples of such salts.

The following schemes 1 and 2 and example 1 describe the processes for the preparation of the compound of formula I in more detail. The starting materials of formulae III, IV and VII are known compounds or may be prepared according to methods known in the art.

In the schemes the following abbreviations have been used:

| | |
|---|---|
| PivCl | pivaloyl chloride |
| THF | tetrahydrofuran |
| TMEDA | N,N,N',N'-tetramethylethylene diamine |
| DIPEA | N-ethyldiisopropyl-amine |

Scheme 1

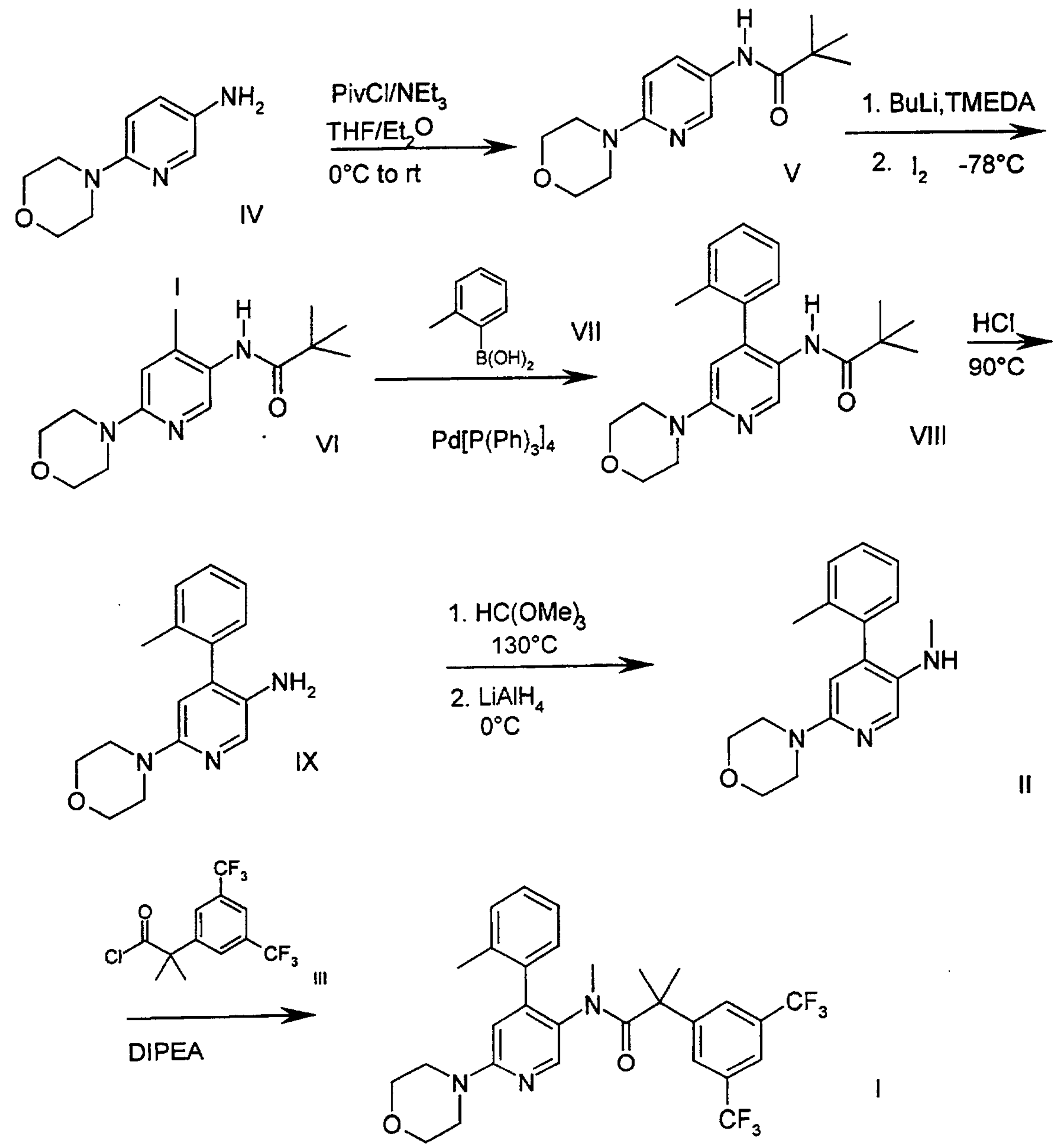
IV
PivCl/NEt3
THF/Et2O
0°C to rt
V
1. BuLi,TMEDA
2. I2 -78°C
VI
VII
Pd[P(Ph)3]4
VIII
HCl
90°C
IX
1. HC(OMe)3
130°C
2. LiAlH4
0°C
II
III
DIPEA
I

Scheme 2

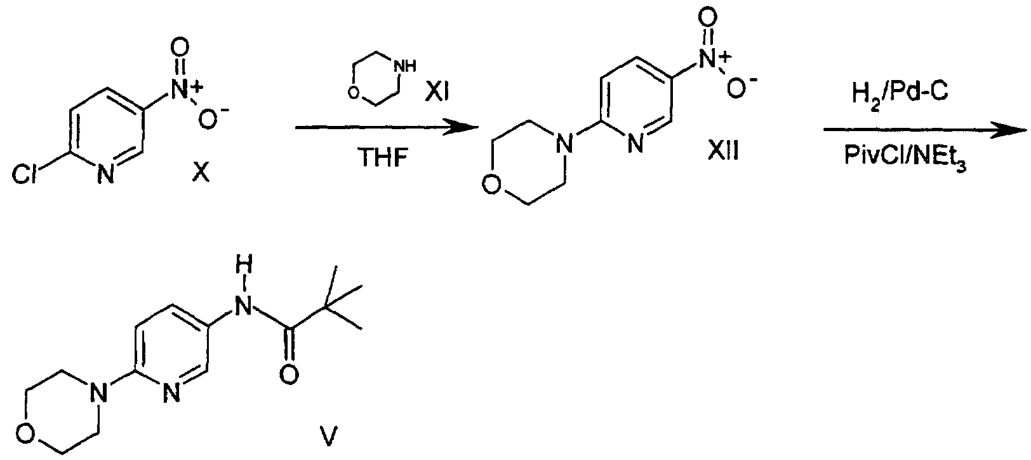


As mentioned earlier, the compound of formula I and its pharmaceutically usable addition salts possess valuable pharmacological properties. It has been found that the compound of the present invention is an antagonist of the Neurokinin 1 (NK-1, substance P) receptor.

The compound of formula I was investigated in accordance with the tests given hereinafter.

The affinity of the compound of formula I for the $NK_1$ receptor was evaluated at human $NK_1$ receptors in CHO cells infected with the human $NK_1$ receptor (using the Semliki virus expression system) and radiolabelled with [$^3$H] substance P (final concentration 0.6 nM). Binding assays were performed in HEPES buffer (50 mM, pH 7.4) containing BSA (0.04 %), leupeptin (8 μg / ml), $MnCl_2$ (3mM) and phosphoramidon (2 μM). Binding assays consisted of 250 μl of membrane suspension (1.25x$10^5$ cells / assay tube), 0.125 μl of buffer of displacing agent and 125 μl of [$^3$H] substance P. Displacement curves were determined with at least seven concentrations of the compound. The assay tubes were incubated for 60 min at room temperature after which time the tube contents were rapidly filtered under vacuum through GF/C filters presoaked for 60 min with PEI (0.3%) with 2 x 2 ml washes of HEPES buffer (50 mM, pH 7.4). The radioactivity retained on the filters was measured by scintillation counting. All assays were performed in triplicate in at least 2 separate experiments.

The compound of formula I is a potent and selective antagonist at recombinant human neurokinin$_1$ ($NK_1$) receptors expressed in CHO cells. It has an affinity (pKi) of 9.0 for the human $NK_1$ receptor over 2 orders of magnitude of selectivity for the $NK_1$ receptor compared to $NK_2$ and $NK_3$ receptors and compared to over 50 other binding sites that have been evaluated.

The activity *in vitro* was examined by studying its effect on substance P induced $Ca^{2+}$ influxes in CHO cells expressing the recombinant human $NK_1$ receptor. In these cells, substance P causes a concentration dependent influx of $Ca^{2+}$ which can be measured using FLIPR technology. Increasing concentrations of 2-(3,5-bis-trifluoromethyl-phenyl) -N-methyl-N-(6-morpholin-4-yl-4-o-tolyl-pyridin-3-yl)-isobutyramide cause a rightward shift in the concentration effect curve of substance P. Expressing these data on a Schild plot allows the calculation of the antagonist affinity ($pA_2$) for this compound of 8.9 (slope of the Schild regression = 1.1). These data indicate that 2-(3,5-bis-trifluoromethyl-phenyl) -N-methyl-N-(6-morpholin-4-yl-4-o-tolyl-pyridin-3-yl)-isobutyramide is a competitive antagonist at human recombinant $NK_1$ receptors.

*In vivo* the compound of formula I antagonises foot-tapping behaviour induced in Gerbils with intracerebroventricular (i.c.v.) injections of an $NK_1$ receptor agonist. The dose for this compound calculated to inhibit 50 % of the foot-tapping behaviour following oral administration was 0.2 mg/kg. The plasma levels required to completely antagonise this behaviour have also been measured and it was found that a total plasma concentration of 10 ng/ml is required to completely block the foot-tapping behavoiur. This antagonism persisted for a number of hours and had a functional half life of 8 hours in this model.

2-(3,5-Bis-trifluoromethyl-phenyl)-N-methyl-N-(6-morpholin-4-yl-4-o-tolyl-pyridin-3-yl)-isobutyramide was also tested as an antiemetic agent in Ferets. Emesis was induced in Ferets by various emetogens (apomorphine, morphine, ipecacuanha, cisplatin and $CuSO_4$. Pretreatment with this compound (0.3 mg/kg, p.o.) 2 hours before the emetigen, completely blocked the emesis induced by all emetogens. A full dose-response curve was constructed against apomorphine-induced emesis and an $ED_{50}$ dose of 0.1 mg/kg, p.o. was calculated.

In a model of motion sickness in the *suncus murinus*, the compound was found to have an $ED_{50}$ of 0.2 mg/kg, p.o.

Therefore, in conclusion, 2-(3,5-bis-trifluoromethyl-phenyl)-N-methyl-N-(6-morpholin-4-yl-4-o-tolyl-pyridin-3-yl)-isobutyramide is a potent antagonist of $NK_1$ induced behaviours in Gerbil and blocks emesis in Ferets and *suncus murinus* with similar potency.

The pharmacokinetic parameters have been evaluated in both rats and dogs. In rats, the compound has a terminal half-life of 23 hours, a clearance of 4 ml/min/kg, a volume of distribution of 8 l/kg and an oral bioavailability of 50 %. In dogs the molecule had a half-life of 40 hours, a clearance of 16 ml/min/kg, a volume of distribution of 22 l/kg and an oral bioavailability of 30-40 %.

The compound of formula I as well as its pharmaceutically usable acid addition salts can be used as medicaments, e.g. in the form of pharmaceutical preparations. The pharmaceutical preparations can be administered orally, e.g. in the form of tablets, coated tablets, dragées, hard and soft gelatine capsules, solutions, emulsions or suspensions. The administration can, however, also be effected rectally, e.g. in the form of suppositories, or parenterally, e.g. in the form of injection solutions.

The compound of formula I and its pharmaceutically usable acid addition salts can be processed with pharmaceutically inert, inorganic or organic excipients for the production of tablets, coated tablets, dragees and hard gelatine capsules. Lactose, corn starch or derivatives thereof, talc, stearic acid or its salts etc can be used as such excipients e.g. for tablets, dragées and hard gelatine capsules.

Suitable excipients for soft gelatine capsules are e.g. vegetable oils, waxes, fats, semisolid and liquid polyols etc.

Suitable excipients for the manufacture of solutions and syrups are e.g. water, polyols, saccharose, invert sugar, glucose etc.

Suitable excipients for injection solutions are e.g. water, alcohols, polyols, glycerol, vegetable oils etc.

Suitable excipients for suppositories are e.g. natural or hardened oils, waxes, fats, semi-liquid or liquid polyols etc.

Moreover, the pharmaceutical preparations can contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

The dosage can vary within wide limits and will, of course, be fitted to the individual requirements in each particular case. In general, in the case of oral administration a daily dosage of about 10 to 1000 mg per person of the compound of formula I should be appropriate, although the above upper limit can also be exceeded when necessary.

The following Example 1 illustrate the present invention without limiting it. All temperatures are given in degrees Celsius.

**Example 1**

**2-(3,5-Bis-trifluoromethyl-phenyl)-N-methyl-N-(6-morpholin-4-yl-4-o-tolyl-pyridin-3-yl)-isobutyramide hydrochloride (1:1.45)**

a) 4-(5-Nitro-2-pyridyl)-morpholine (XII)

To a solution of 20 g (126 mmol) of 2-chloro-5-nitropyridine in 150 ml tetrahydrofuran were added dropwise 27 ml (315 mmol) morpholine within 10 min. The reaction mixture was refluxed for additional 2h. After cooling to room temperature, the solvent was removed *in vacuo* and the residue was re-dissolved in 200 ml ethyl acetate. The organic phase was washed with 200 ml 1 N sodium bicarbonate solution, dried (magnesium sulfate) and evaporated to give 27.3 g (quantitative) of the title compound as a yellow solid. M.p. 142-143°C.

b) 2,2-Dimethyl-N-(6-morpholin-4-yl-pyridin-3-yl)-propionamide (V)

To a solution of 27.3 g (126 mmol) of4-(5-nitro-2-pyridyl)-morpholine in 600 ml methanol were added 2.5 g of 10 % of palladium on activated charcoal. The reaction mixture was hydrogenated (room temperature to *ca*. 45°C, 1 bar) until the theoretical amount of hydrogen was taken up (about 3h). The catalyst was filtered off and was washed twice with 100 ml portions of methanol. The filtrate was evaporated *in vacuo* to give 22.6 g of a purple oil which consisted to *ca*. 95 % of the desired aniline derivative according to analysis by thin layer chromatography.

This crude product was dissolved in a mixture of 240 ml tetrahydrofuran and 60 ml diethyl ether. After cooling to 0°C, 26 ml (189 mmol) of triethylamine were added in one portion. Stirring was continued while 23 g (189 mmol) of pivaloyl chloride were added dropwise within a period of 10 min. The ice bath was removed and the reaction mixture was stirred for 1h at room temperature. Then, the solvent was removed *in vacuo* and the residue was suspended in 200 ml 1 N sodium bicarbonate solution. The product was extracted three times with 200 ml portions of dichloromethane,

dried (sodium sulfate) and evaporated. Recrystallization of the solid residue from ethyl acetate/hexane 1:8 gave 28.6 g (86%) of the title compound as white crystals.

MS m/e (%): 264 ($M+H^+$, 100).

c) N-(4-Iodo-6-morpholin-4-yl-pyridin-3-yl)-2,2-dimethyl-propionamide (VI)

A solution of 28.4 g (108 mmol) 2,2-dimethyl-N-(6-morpholin-4-yl-pyridin-3-yl)-propionamide and 49 ml (324 mmol) N,N,N',N'-tetramethylethylenediamine under argon in 600 ml tetrahydrofuran was cooled in a dry ice bath to -78°C. Within 1h, 202 ml (324 mmol) of a 1.6 N n-butyllithium solution in hexane were added dropwise. The reaction mixture was allowed to warm up to -35°C overnight. After cooling again to -78°C, 37 g (146 mmol) iodine dissolved in 60 ml tetrahydrofuran were added dropwise during 15 min. The dry ice bath was replaced by an ice bath and a solution of 90 g (363 mmol) sodium thiosulfate pentahydrate in 250 ml water were added within 10 min when the temperature of the reaction mixture had reached 0°C. Then, 1000 ml diethyl ether were added and the organic layer was separated. The aqueous layer was extracted twice with 500 ml dichloromethane and the combined organic layers were dried (magnesium sulfate) and evaporated. Flash chromatography gave 15.6 g (37%) of the title compound as a light brown oil which crystallized upon standing at room temperature.
MS m/e (%): 389 ($M^+$, 71), 358 (25), 304 (43), 57 (100).

d) 2,2-Dimethyl-N-(6-morpholin-4-yl-4-o-tolyl-pyridin-3-yl)-propionamide (VIII)

A mixture of 3.50 g (9.0 mmol) N-(4-iodo-6-morpholin-4-yl-pyridin-3-yl)-2,2-dimethylpropionamide, 35 ml toluene, 18 ml 2 N sodium carbonate solution, 312 mg (0.27 mmol) tetrakis(triphenylphosphine)palladium(0) and 1.34 g (9.9 mmol) o-tolylboronic acid was heated under argon at 80°C for 12h. After cooling to room temperature, the aqueous phase was separated and washed twice with ethyl acetate. The combined organic layers were washed with 50 ml brine, dried (sodium sulfate) and evaporated. Purification by flash-chromatography gave 3.23 g (quantitative) of the title compound as a white foam.
MS m/e (%): 354 ($M+H^+$, 100).

e) 6-Morpholin-4-yl-4-o-tolyl-pyridin-3-ylamine (IX)

A suspension of 2.93 g (8.28 mmol) 2,2-dimethyl-N-(6-morpholin-4-yl-4-o-tolyl-pyridin-3-yl)-propionamide in 80 ml 3 N hydrochloric acid solution and 5 ml 1-propanol was heated to 90-95°C overnight. The reaction mixture was cooled to room temperature, washed with three 20 ml portions diethyl ether and filtered over celite. The filtrate was diluted with 20 ml water and was adjusted to pH 7-8 by addition of 28 % sodium hydroxide solution under ice cooling. The product was extracted with four 100 ml portions of dichloromethane. The combined organic layers were washed with 50 ml brine, dried (magnesium sulfate) and evaporated to give 2.31 g (quantitative) of the title compound as a white foam.
MS m/e (%): 269 ($M^+$, 100).

f) Methyl-(6-morpholin-4-yl-4-o-tolyl-pyridin-3-yl)-amine (II)

A solution of 2.24 g (8.3 mmol) 6-morpholin-4-yl-4-o-tolyl-pyridin-3-ylamine in 17 ml trimethyl orthoformate and 3 drops trifluoroacetic acid was heated for 2h at 130°C. The reaction mixture was evaporated and dried *in vacuo* for 30 min. The residual oil was dissolved in 5 ml tetrahydrofuran and was added dropwise under ice cooling to 630 mg (16.6 mmol) lithium aluminum hydride in 20 ml tetrahydrofuran. The reaction mixture was stirred for 1h at room temperature, cooled to 0°C again and acidified (pH 1-2) by addition of 28 % hydrochloric acid solution. After stirring for 5 min, 28 % sodium hydroxide solution was added to reach pH 10. The solution was filtered over celite, evaporated and purified by flash chromatography to give 1.56 g (66%) of the title compound as a white foam.
MS m/e (%): 283 ($M^+$, 100).

g) 2-(3,5-Bis-trifluoromethyl-phenyl)-N-methyl-N-(6-morpholin-4-yl-4-o-tolyl-pyridin-3-yl)-isobutyramide (I)

A solution of 1.46 g (5.15 mmol) methyl-(6-morpholin-4-yl-4-o-tolyl-pyridin-3-yl)-amine and 1.32 ml (7.73 mmol) N-ethyldiisopropylamine in 15 ml dichloromethane was cooled in an ice bath and 1.8 g (5.67 mmol) 2-(3,5-bis-trifluoromethyl-phenyl)-2-methylpropionyl chloride were added dropwise. The reaction mixture was warmed to 35-40°C for 3h, cooled to room temperature again and was stirred with 25 ml saturated sodium bicarbonate solution. The organic

layer was separated and the aqueous phase was extracted with dichloromethane. The combined organic layers were dried (magnesium sulfate) and evaporated. The residue was purified by flash chromatography to give 2.9 g (quantitative) of the title compound as white crystals. M.p. 131-132°C.

h) 2-(3,5-Bis-trifluoromethyl-phenyl)-N-methyl-N-(6-morpholin-4-yl-4-o-tolyl-pyridin-3-yl)-isobutyramide hydrochloride (1:1.45)

To a solution of 2.9 g (5.13 mmol) 2-(3,5-bis-trifluoromethyl-phenyl)-N-methyl-N-(6-morpholin-4-yl-4-o-tolyl-pyridin-3-yl)-isobutyramide in 50 ml diethyl ether were added under ice cooling 2.8 ml 3 N hydrochloric acid solution in diethyl ether. After stirring for 15 min at 0°C, the suspension was evaporated to dryness, re-suspended in 100 ml diethyl ether, filtered and dried *in vacuo* to give 2.82 g (89%) of the title compound as white crystals.
MS m/e (%): 566 ($M+H^+$, 100), 588 ($M+Na^+$, 11).

Example A

Tablets of the following composition are manufactured in the usual manner:

| | mg/tablet | |
|---|---|---|
| Active substance | | 5 |
| Lactose | | 45 |
| Corn starch | | 15 |
| Microcrystalline cellulose | | 34 |
| Magnesium stearate | | 1 |
| | Tablet weight | 100 |

Example B

Capsules of the following composition are manufactured:

| | mg/capsule | |
|---|---|---|
| Active substance | | 10 |
| Lactose | | 155 |
| Corn starch | | 30 |
| Talc | | 5 |
| | Capsule fill weight | 200 |

The active substance, lactose and corn starch are firstly mixed in a mixer and then in a comminuting machine. The mixture is returned to the mixer, the talc is added thereto and mixed thoroughly. The mixture is filled by machine into hard gelatine capsules.

Example C

Suppositories of the following composition are manufactured:

| | mg/supp. | |
|---|---|---|
| Active substance | | 15 |
| Suppository mass | | 1285 |
| | Total | 1300 |

The suppository mass is melted in a glass or steel vessel, mixed thoroughly and cooled to 45°C. Thereupon, the finely powdered active substance is added thereto and stirred until it has dispersed completely. The mixture is poured into suppository moulds of suitable size, left to cool, the suppositories are then removed from the moulds and packed individually in wax paper or metal foil.

## Claims

1. The compound of the formula

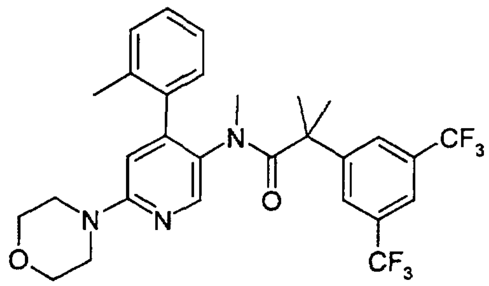

I

and to pharmaceutically acceptable acid addition salts thereof.

2. The compound according to claim 1, which is 2-(3,5-bis-trifluoromethyl-phenyl)-N-methyl-N-(6-morpholin-4-yl-4-o-tolyl-pyridin-3-yl)-isobutyramide.

3. A medicament containing the compound as claimed in claims 1 and 2 and pharmaceutically acceptable excipients.

4. A medicament according to claim 3 for the treatment of diseases related to the NK-1 receptor.

5. A process for preparing the compound of formula I as defined in claim 1, which process comprises

a) reacting a compound of formula

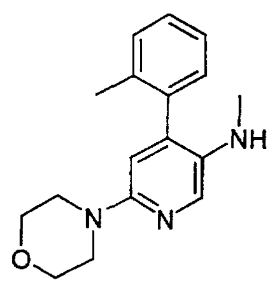

II

with a compound of formula

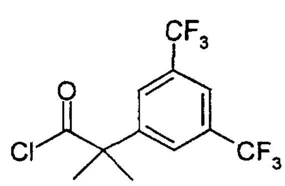

III

to a compound of formula

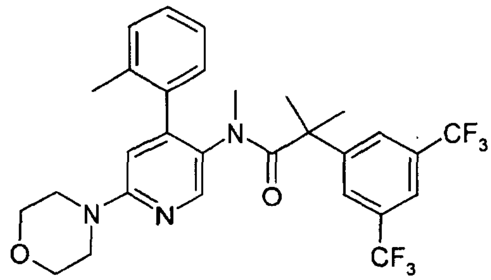

I

and,
if desired, converting the compound obtained into a pharmaceutically acceptable acid addition salt.

**6.** The use of the compound of formula I in any one of claims 1 and 2 for the manufacture of medicaments for the treatment of diseases relating to the NK-1 receptor.

**7.** The use of the compound of formula I in accordance to claim 6 for the manufacture of medicaments for the treatment of depression, anxiety or emesis.

**Patentansprüche**

**1.** Verbindung der Formel

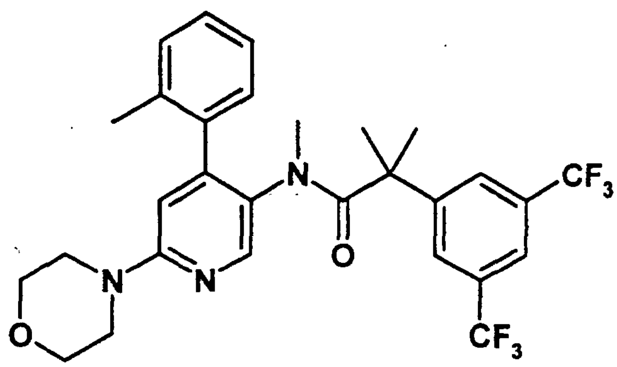

I

und pharmazeutisch annehmbare Säureadditionssalze davon.

**2.** Verbindung nach Anspruch 1, welche 2-(3,5-Bis-trifluormethyl-phenyl)-N-methyl-N-(6-morpholin-4-yl-4-o-tolyl-pyridin-3-yl)-isobutyramid ist.

**3.** Medikament enthaltend die Verbindung wie in den Ansprüchen 1 und 2 beansprucht und pharmazeutisch annehmbare Arzneistoffträger.

**4.** Medikament nach Anspruch 3 zur Behandlung von Krankheiten, die mit dem NK-1-Rezeptor zusammenhängen.

**5.** Verfahren zur Herstellung der Verbindung der Formel I, wie sie in Anspruch 1 definiert ist, welches umfaßt

a) Umsetzen einer Verbindung der Formel

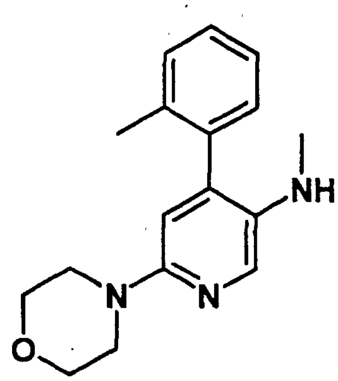

II

mit einer Verbindung der Formel

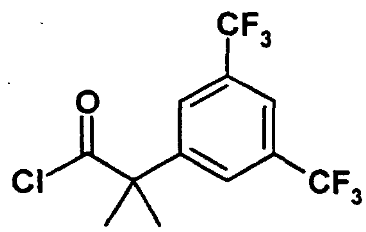

III

zu einer Verbindung der Formel

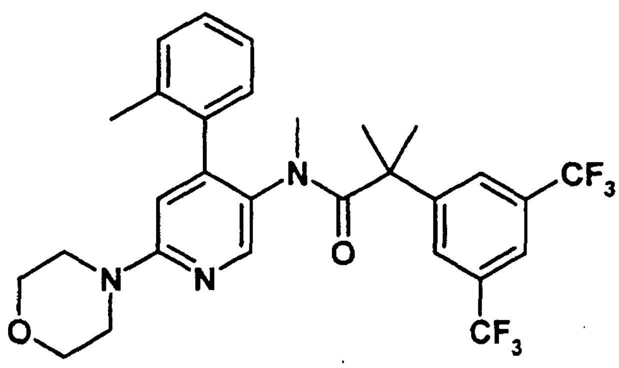

I

und,
wenn gewünscht, Überführen der erhaltenen Verbindung in ein pharmazeutisch annehmbares Säureadditionssalz.

**6.** Verwendung der Verbindung der Formel I aus einem der Ansprüche 1 und 2 zur Herstellung eines Medikaments zur Behandlung von Krankheiten, die mit dem NK-1-Rezeptor zusammenhängen.

**7.** Verwendung der Verbindung der Formel I gemäß Anspruch 6 zur Herstellung von Medikamenten zur Behandlung von Depression, Angst oder Emesis.

## Revendications

1. Composé de formule

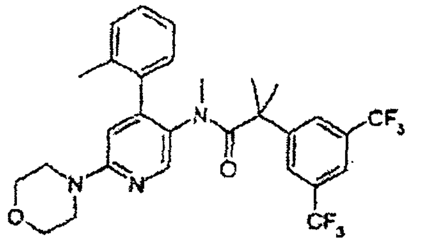

I

et les sels d'addition acides pharmaceutiquement acceptables de ceux-ci.

2. Composé selon la revendication 1, qui est le 2-(3,5-bis-trifluorométhyl-phényl)-N-méthyl-N-(6-morpholin-4-yl-4-o-tolyl-pyridin-3-yl)-isobutyramide.

3. Médicament contenant le composé selon les revendications 1 et 2 et des excipients pharmaceutiquement acceptables.

4. Médicament selon la revendication 3 pour le traitement des maladies associées au récepteur NK-1.

5. Procédé pour préparer le composé de formule I selon la revendication 1, lequel procédé comprend :

a) la réaction d'un composé de formule

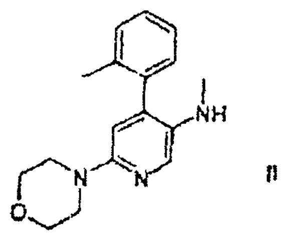

II

avec un composé de formule

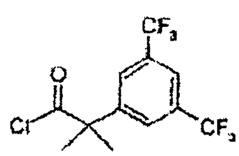

III

en un composé de formule

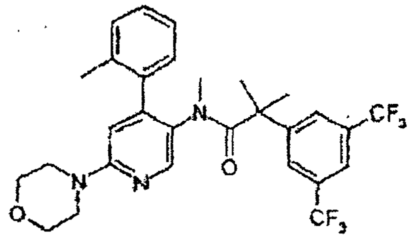

et,

si on le souhaite, la conversion du composé obtenu en un sel d'addition acide pharmaceutiquement acceptable.

6. Utilisation du composé de formule I selon l'une quelconque des revendications 1 et 2 pour la fabrication de médicaments pour le traitement de maladies concernant le récepteur NK-1.

7. Utilisation du composé de formule I selon la revendication 6 pour la fabrication de médicaments pour le traitement de la dépression, de l'anxiété ou de l'émèse.